# EUROPEAN PATENT SPECIFICATION

(11) **EP 2 613 693 B1**
(45) Date of publication and mention of the grant of the patent: **20.04.2016**
(21) Application number: 11840226.2
(22) Date of filing: 09.11.2011
(51) Int. Cl.: A61B 5/042

(54) **ELECTROPHYSIOLOGY CATHETERS HAVING A LOOP**
ELEKTROPHYSIOLOGISCHE KATHETER MIT EINEM SCHLINGENTEIL
CATHÉTERS D'ÉLECTROPHYSIOLOGIE AYANT UNE PARTIE DE BOUCLE

(30) Priority: 09.11.2010 US 411694 P; 30.12.2010 US 98262910; 30.12.2010 US 98261810
(43) Date of publication of application: 17.07.2013
(73) Proprietor: St. Jude Medical, Atrial Fibrillation Division, Inc., St. Paul, Minnesota 55117-9913 (US)
(72) Inventor: AFONSO, Valtino X., Oakdale Minnesota 55128-5543 (US); POTTER, Daniel J., Stillwater Minnesota 55082 (US); CHAN, Howard, Plymouth Minnesota 55441 (US); ROY, Pianka, Irvine California 92620 (US); DE LA RAMA, Alan, Cerritos California 90703 (US); VELASCO, Jennifer, Orange California 92865 (US); CHEN, Kailing, Laguna Hills California 92656 (US)
(74) Representative: Kramer Barske Schmidtchen Patentanwälte PartG mbB
(86) International application number: PCT/US2011/059922
(87) International publication number: WO 2012/064818

(56) References cited:
- WO-A1-2010/048676
- WO-A2-2006/044794
- US-A1- 2001 007 070
- US-A1- 2003 088 244
- US-A1- 2004 147 827
- US-A1- 2005 187 455
- US-A1- 2007 270 679
- US-A1- 2007 270 679
- US-A1- 2009 062 789
- US-A1- 2009 163 794
- US-A1- 2010 069 733

## Description

### BACKGROUND OF THE INVENTION

### a. Field of the Invention

The disclosure relates to electrophysiology (EP) catheters for use in medical procedures. In particular, the disclosure relates to a family of catheters for use in diagnostic and therapeutic procedures in and around a patient's cardiac anatomy, such as the ostium of a pulmonary vein.

### b. Background Art

Catheters are used for an ever-growing number of procedures. For example, catheters are used for diagnostic, therapeutic, and ablative procedures, to name just a few examples. Typically, the catheter is manipulated through the patient's vasculature and to the intended site, for example, a site within the patient's heart.

A typical EP catheter includes an elongate shaft and one or more electrodes on the distal end of the shaft. The electrodes can be used for diagnosis, mapping, ablation, and/or the like. Oftentimes, these electrodes are ring electrodes that extend about the entire circumference of the catheter shaft.

One specific use of an EP catheter is to map the atrial regions of the heart, and in particular the pulmonary veins, which are often origination points or foci of atrial fibrillation. Such EP mapping catheters typically have at least a partial loop shape at their distal end in order to surround the pulmonary vein ostia. Because of varying patient anatomies, however, it can be challenging to properly maneuver and place the looped section of the catheter precisely in the pulmonary vein ostia.
US 2003/088244 A1 relates to a device for ablating body tissue comprising a support element having a curved region to peripherally contact a tissue area, and at least two energy emitting zones on the curved region mutually separated across the contacted tissue area, the mutual separation between the zones across the contacted tissue area creating, when the zones simultaneously emit energy, an additive heating effect to form a continuous lesion pattern in the contacted tissue area that spans across the contacted tissue area.
WO 2010/048676 A1 relates to a catheter assembly comprising a first elongate tubular member having a proximal end portion defining a proximal end, a distal end portion having an opening therein and defining a distal end, and at least one lumen defined between the proximal end and the distal end, a second tubular member having a proximal end portion defining a proximal end, a distal end portion defining a distal end, and at least one lumen defined between the proximal end and the distal end, the second elongate tubular member being received within the at least one lumen of the first elongate tubular member, such that the distal end portion of the second elongate tubular member projects from the opening in the distal end portion of the first elongate tubular member, and an elongate, shape-imparting element receivable in the at least one lumen of at least one of the first elongate tubular member and the second tubular member, the shape-imparting element imparting a non-rectilinear shape to the distal end portion of at least one of the first elongate tubular member and the second elongate tubular member.
WO 2006/044794 A2 discloses an ablation catheter, comprising (a) an elongated, flexible, tubular body member having a proximal end, a distal end and a lumen extending therebetween, (b) a control shaft coaxially disposed and slidingly received within the lumen of the tubular body member, (c) a flexible carrier assembly which includes at least one ablation element used to deliver energy to tissue, and (d) a coupler which connects the control shaft to the carrier assembly, wherein retraction of the control shaft causes the carrier assembly to be constrained within the lumen of the tubular body member, and wherein advancement of the control shaft causes the carrier assembly to extend beyond the distal end of the tubular member body.
US 2007/0270679 A1 relates to a catheter comprising a proximal section comprising a shaft and a control section at a proximal end, the control section operably connected to two or more bidirectional control wires and a variable radius control wire, and a distal section comprising at desired points along its length at least one electrode, a connection point for the variable radius control wire, and a connection point for the at least two bidirectional control wires, wherein engaging one or more of the bidirectional control wires causes a left to right motion in the distal section, and wherein engaging the variable radius control wire modifies the radius of the loop.

### BRIEF SUMMARY OF THE INVENTION

It is desirable to be able to provide a family of EP catheters having highly enhanced deflection capability to effectively access a particular subject's individual anatomy while at the same time rapidly collecting EP diagnostic data from the subject with a cardiac mapping portion comprising a loop. In this way, the family of EP catheters designed, built, and/or implemented in accordance with the present disclosure can be advantageous because the distal portion thereof (*i.e.,* the cardiac mapping portion) can be efficiently deflected relative to the remainder of the catheter body, and thus, can efficiently map various surfaces of the heart via the plurality of electrodes (*e.g.,* about 10 or 20 or any other number) disposed on the cardiac mapping portion.

For example and without limitation, EP catheters in accordance with the present disclosure can provide for about 180 degree uni-directional deflection with a small turning radius that allows physicians to more easily maneuver the EP catheters to desired locations in different subject anatomies, including hard-to-reach areas such as the ostium of the right interior pulmonary vein. The construction of the EP catheters in accordance with the present disclosure can include an extended braid in some embodiments which provides physicians with responsive torque transfer of the catheter body. In addition, the number of electrodes disposed on the cardiac mapping portion of the EP catheters can allow for faster and denser electroanatomical mapping with a single EP catheter. According to the present invention, at least a dual loop cardiac mapping portion including an outer loop of variable diameter or radius of curvature and at least one inner loop of fixed diameter or radius of curvature may enable multiple diagnostic functions, including facilitating both pulmonary vein isolation assessment with the outer loop of variable diameter and high density mapping with both the outer loop and inner loop(s). Although at least one inner loop is described as being of fixed diameter, at least one of the inner loops can be of variable diameter or radius in accordance with other embodiments of the disclosure. For example and without limitation, the adjustability of the diameter or radius of the outer loop and at least one of the inner loops can be tandem in some embodiments of the disclosure. For another example and without limitation, the adjustability of the diameter or radius of the outer loop and at least one of the inner loops can be independent of each other in some embodiments of the disclosure.

In accordance with the present invention, the EP catheter has a proximal region, a neck region coupled to the proximal region, and a distal portion coupled to the neck region. The distal portion is predisposed into a plurality of loops when in a non-compressed state. A majority of each of the plurality of the loops is disposed within a common plane that is perpendicular or substantially perpendicular to the longitudinal axis of the EP catheter. An axis of each of the plurality of loops can be substantially the same as the longitudinal axis of the EP catheter. The EP catheter can include a braided structure embedded near a junction of the neck region and the distal portion.

At least a portion of the neck region can reside substantially along the longitudinal axis of the EP catheter. The distal portion can have an outermost diameter between about 15 mm and about 40 mm. For example and without limitation, the distal portion can have an outer diameter of about 20 mm. Although these dimensions are mentioned in detail, other dimensions are not excluded.

The distal portion can be configured to deflect in a circular turn at least about 180 degrees relative to the proximal region of the catheter body when the distal portion is in a non-compressed state. By allowing the distal portion to be deflected relative to the remainder of the catheter body, the angle of the distal portion can be fine tuned. A radius of the circular turn can be no more than 50 mm in some embodiments of the disclosure. Enabling the deflection of at least about 180 degrees in such a small space can be ideal for performing cardiac diagnostic mapping of the atria, for example. In accordance with the present invention, an outermost loop of the plurality of loops has a variable diameter, and an innermost loop of the plurality of loops has a fixed diameter.

The distal portion can include a shape memory material comprising a nickel titanium material or alloy thereof in accordance with some embodiments of the disclosure. The shape memory material can be configured to shape at least a portion of the distal portion into the plurality of loops disposed within a common plane, each of the plurality of loops having a predefined radius of curvature. The outer diameter of the catheter body can vary. For example and without limitation, a majority of the catheter body, the proximal region, can be on the order of about 7F, and an adjacent neck region can include structure, including an anchoring location for an activation wire that transitions the outer diameter to about 4F such that the distal portion is about 4F or other uniform diameter throughout. Accordingly, an outer diameter of the distal portion (*e.g*., about 4F) can be less than an outer diameter of the proximal region (*e.g*., about 7F).

The EP catheter can include a plurality of electrodes coupled to the distal portion. In accordance with some embodiments of the disclosure, the distal portion includes about 10 to about 20 discrete electrodes, also known as deca and duo-deca pole or polar electrode arrangements, with unipolar and bipolar pairing provided via suitable switching, as desired. In accordance with some embodiments of the disclosure, at least one of the plurality of electrodes can be coupled to a distal tip portion of the loop. The distal tip electrode can be relatively longer in accordance with some embodiments of the disclosure. For example and without limitation, the tip electrode can have a length of between about 1 mm and about 4 mm, and the remainder of the electrodes can have a length of about 1 mm. In those embodiments where the loop of the distal portion includes about 20 electrodes, the distal portion can include about 19 discrete ring-type electrodes and a single distal tip electrode. In one embodiment of the disclosure, the 20 electrodes may be substantially evenly spaced on the distal portion (*e.g*., about 3 mm, about 4 mm, about 5 mm, about 7 mm, or the like). For example and without limitation, about 20 discrete electrodes can be substantially evenly spaced about 4 mm apart if the outermost diameter of the plurality of loops is about 20 mm. For another example and without limitation, the 20 discrete electrodes can be spaced about 6.5 mm apart. In accordance with other embodiments of the disclosure, the about 20 electrodes can be distributed in a paired bi-polar mapping configuration wherein each pair is equally separated from each other pair (*e.g*., about 2.5 mm to about 7 mm apart) and the electrodes of each individual pair are closely situated (*e.g*., about 1 mm apart to about 2.5 mm apart-including the tip electrode to the most distal ring-type electrode). For example and without limitation, a 1-2.5-1 arrangement can be used on a plurality of loops with the outermost of the plurality of loops having an outermost diameter of about 20 mm. Although these particular spacing configurations are mentioned in detail, the spacing may be greater or less in other embodiments of the disclosure.

The EP catheter can further comprise a handle joined to the proximal region for controlling deflection of the distal portion of the catheter body and an activation wire extending through at least a portion of the proximal region of the catheter body. The activation wire can be configured to deflect the neck region of the catheter body in a common plane. Alternatively, the activation wire can be configured to deflect the proximal region, the neck region, and/or the distal portion of the catheter body in different planes. The handle can include a means for imparting tension to the activation wire such that forces acting on the activation wire are transmitted to the proximal region in order to deflect the catheter body, thereby deflecting the distal portion up to about 180 degrees. In general, the activation wire can be coupled to a first element (*e.g.*, a round or flat wire, a thread of fiber such as a para-aramid synthetic fiber including those sold under the brand name KEVLAR® available from E. I. du Pont de Nemours and Company, or the like) such that forces are transferred to the catheter shaft proximal of the loop (or the neck region) via an actuation mechanism, such as a rotary knob or push-pull handle as is known in the EP art. The means for imparting tension to the activation wire and/or the first element can comprise a flat wire subassembly disposed between the handle and the distal portion in accordance with an embodiment of the disclosure. The handle can further include a mass electrical termination structure for receiving a plurality of electrical conductors. The EP catheter further includes at least one first tension element coupled to at least one anchor member within the distal portion and coupled to an actuator in the handle for altering a diameter of the outermost loop of the plurality of loops of the distal portion. In various embodiments, at least one tension element may also or alternatively be coupled to at least one in the distal portion and coupled to an actuator in the handle for altering a diameter of a non-outermost loop, such as the innermost loop, of the plurality of loops. Accordingly, the EP catheter can further include at least one second tension element coupled to at least one anchor member within the at least one of the inner loops of the distal portion and coupled to an actuator in the handle for altering a diameter of at least one of the inner loops of the plurality of loops of the distal portion in accordance with some embodiments of the disclosure. Further, in at least one embodiment, a first tension element may be coupled to a first anchor member within the distal portion and coupled to a first actuator within the handle for altering a diameter of the outermost loop of the plurality of loops in the distal portion, and a second tension element may be coupled to a second anchor member within the distal portion and coupled to a second actuator within the handle for altering a diameter of the innermost loop of the plurality of loops in the distal portion. The outermost loop of the plurality of loops can include a plurality of electrodes and the innermost loop of the plurality of loops can include a plurality electrodes. The EP catheter can include a switch mechanism for controlling whether at least one of the plurality of electrodes on the outermost loop is activated and whether at least one of the plurality of electrodes on the innermost loop is activated.

The foregoing and other aspects, features, details, utilities, and advantages of the present invention will be apparent from reading the following description and claims, and from reviewing the accompanying drawings.

### BRIEF DESCRIPTION OF THE DRAWINGS

FIG. 1A is a plan view including a partially exploded depiction of an exemplary EP catheter having a distal portion, with EP electrodes disposed in a preconfigured manner, and with the partially exploded depiction illustrating the catheter in both a deflected and an undeflected configuration.
FIG. 1B is a plan view of the exemplary EP catheter illustrated in FIG. 1A in an undeflected configuration.
FIG. 2A is a plan view including a partially exploded depiction of an exemplary EP catheter having a distal portion in accordance with an embodiment of the disclosure, with EP electrodes disposed in a preconfigured manner, and with the partially exploded depiction illustrating the catheter in both a deflected and an undeflected configuration.
FIG. 2B is a plan view of the exemplary EP catheter illustrated in FIG. 2A in an undeflected configuration.
FIG. 2C depicts one configuration for varying the radius of curvature of the distal portion of the exemplary EP catheter depicted in FIGS. 2A-2B in accordance with an embodiment of the disclosure.
FIG. 2D is a diagrammatic representation of an exemplary EP catheter in accordance with at least one embodiment of the disclosure.
FIG. 3A is an enlarged view of the cardiac mapping portion of the exemplary EP catheters of FIGS. 1A and 2A, namely, an illustration of a pair of electrodes residing on a segment of the distal portion.
FIG. 3B is an elevational side view in partial cross section of a neck portion formed just proximal of the distal portions of the exemplary EP catheters depicted in FIGS. 1A, 1B, 2A, and 2B.
FIG. 4A is a close up isometric view of the distal portion of the exemplary EP catheter of FIGS. 1A and 1B (with a view of connecting elements within interior portions of the catheter body illustrated).
FIG. 4B is a close up isometric view of the distal portion of the exemplary EP catheter of FIGS. 2A and 2B in accordance with an embodiment of the disclosure (with a view of connecting elements within interior portions of the catheter body illustrated).
FIG. 4C is an enlarged isometric fragmented view of the interior details of the ends of the connecting elements within the interior of the catheter bodies shown in FIGS. 4A-4B.
FIG. 4D is an enlarged fragmented plan view of the interior details of the ends of the connecting elements within the interior of the catheter bodies shown in FIGS. 4A-4B, including a plan view of a polymer tube having a flattened end to promote adhesive effectiveness at its proximal end to a flat wire subassembly that is used to contain a segment of nitinol shape memory wire that forms the distal portion of the EP catheters.
FIG. 5A is an elevational view showing exemplary dimensions of the distal portion of the exemplary EP catheter of FIGS. 1A and 1B. At the perspective illustrated, the off-axis junction with the catheter body is not apparent.
FIG. 5B is an elevational view showing exemplary dimensions of the distal portion of the exemplary EP catheter of FIGS. 2A and 2B according to an embodiment of the disclosure.
FIG. 6A depicts the distal portion of the exemplary EP catheter of FIGS. 1A and 1B (with cross-references to details shown in FIGS. 6C-6D).
FIG. 6B depicts the distal portion of the exemplary EP catheter of FIGS. 2A and 2B (with cross-reference to details shown in FIG. 6C-6D).
FIG. 6C is an enlarged fragmentary view in partial cross section and partial cut-away of the distal tip electrode and two ring electrodes and flat wire subassembly connection within the catheter body, respectively, shown in FIG. 6A or 6B.
FIG. 6D is an enlarged cross sectional view of the catheter body near the neck region shown in FIGS. 6A and 6B.
FIG. 7 is a cross-sectional view of the EP catheter illustrated in FIG. 6D taken along line 7-7 as shown in FIG. 6D.

### DETAILED DESCRIPTION OF THE INVENTION

For example and without limitation, the present teachings will be described with reference to EP catheters utilized in cardiac EP studies, such as the AFOCUS II™ and/or AFOCUS II EB™ diagnostic catheters of St. Jude Medical, Atrial Fibrillation Division, Inc., which can provide access to difficult-to-reach portions of atrial anatomy, such as the right superior and inferior pulmonary veins. The EP catheters described herein can also provide relatively faster cardiac activity data collection having the necessary detail to efficiently diagnose complex cardiac arrhythmias, including, for example, in duodecapolar configurations. It should be understood, however, that the present teachings can be applied to good advantage in other contexts as well, such as radiofrequency (RF) ablation catheters or other diagnostic cardiac catheters. In use, EP catheter 10 can be introduced into a patient's body proximate an area of interest, such as a pulmonary vein ostium. Of course, EP catheter 10 can be introduced surgically (*e.g*., via an incision in the patient's chest) or non-surgically (*e.g.,* navigated through the patient's vasculature to a desired site). The EP catheter 10 can be operated manually by a clinician or via a clinician-surrogate, such as a computer processor-controlled surgical system.

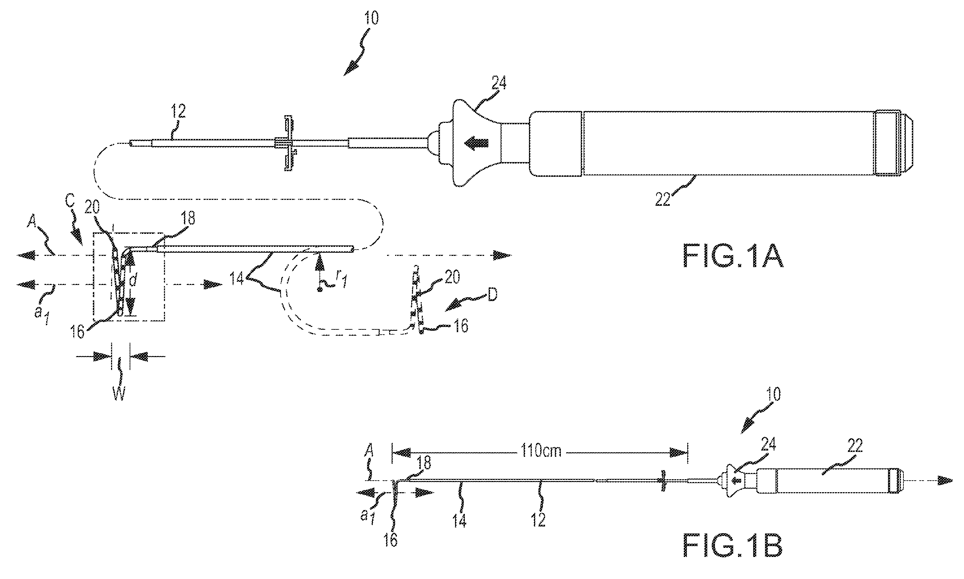

Referring now to the drawings, FIGS. 1A and 1B depict an EP catheter 10. FIG. 1A is a plan view including a partially exploded depiction of an exemplary EP catheter 10 having an elongate catheter body 12 and a distal portion 16 comprising a cardiac mapping portion with EP diagnostic, or mapping, electrodes 20 (as depicted herein arranged in an exemplary decapolar configuration), with the partially exploded depiction illustrating the catheter 10 in both an undeflected and a deflected configuration (denoted as "C" and "D" respectively). FIG. 1B is a plan view of the exemplary EP catheter 10 illustrated in FIG. 1A in an undeflected configuration (*i.e*., configuration "C" of FIG. 1 A).

The elongate catheter body 12 of EP catheter 10 has a longitudinal axis A. The catheter body 12 of EP catheter 10 can be tubular (*e.g.,* it defines at least one lumen therethrough). The catheter body 12 includes a proximal region 14, a distal portion 16, and a neck region 18 between proximal region 14 and distal portion 16. The neck region 18 is coupled to the proximal region 14, and the distal portion 16 is coupled to the neck region 18. FIG. 1B shows an approximate minimum length for the catheter body 12 on the order of about 110 cm, although other lengths can be employed according to various embodiments of this disclosure. The overall length of catheter body 12 should generally be long enough to reach the intended destination within the patient's body. One of ordinary skill in the art will appreciate that the relative lengths of proximal region 14, distal portion 16, and neck region 18 depicted in FIGS. 1A and 1B are merely illustrative and can vary without departing from the scope of the present invention. Preferably, the combined length of proximal region 14, distal portion 16, and neck region 18 should not be less than about 110 cm.

Catheter body 12 can typically be made of a biocompatible polymeric material, such as polytetrafluoroethylene (PTFE) tubing (*e.g*., TEFLON® brand tubing). Of course, other polymeric materials, such as fluorinated ethylene-propylene copolymer (FEP), perfluoroalkoxyethylene (PFA), poly(vinylidene fluoride), poly(ethylene-co-tetrafluoroethylene), and other fluoropolymers, can be utilized. Additional suitable materials for catheter body 12 include, without limitation, polyamide-based thermoplastic elastomers (namely poly(ether-block-amide), such as PEBAX®), polyester-based thermoplastic elastomers (*e.g.,* HYTREL®)., thermoplastic polyurethanes (*e.g.,* PELLETHANE®, ESTANE®), ionic thermoplastic elastomers, functionalized thermoplastic olefins, and any combinations thereof. In general, suitable materials for catheter body 12 can also be selected from various thermoplastics, including, without limitation, polyamides, polyurethanes, polyesters, functionalized polyolefins, polycarbonate, polysulfones, polyimides, polyketones, liquid crystal polymers and any combination thereof. It is also contemplated that the durometer of catheter body 12 can vary along its length. In general, the basic construction of catheter body 12 will be familiar to those of ordinary skill in the art, and thus will not be discussed in further detail herein. In addition, a variety of localization, visualization, and/or orientation-specific elements can be incorporated into the devices described, depicted, and/or claimed herein. For example and without limitation, metallic coil members, impedance based electrodes, fluoroscopically opaque materials, and the like may be incorporated into the devices described, depicted, and/or claimed herein for use in conjunction with an electroanatomical localization and visualization system, for example.

The distal portion 16 can be located on a distal end of the EP catheter 10. The distal portion 16 can be predisposed into a loop consisting of a single, helical revolution about the axis *a₁* when in a non-compressed state. The loop can have a fixed diameter. The loop can be generally circular. While the loop is described as generally circular, it may take any number of shapes, for example, elliptical. While the distal portion 16 is shown as forming a single revolution, the distal portion 16 may include a plurality of revolutions to define a spiral or coil. The distal portion 16 can be helical as generally illustrated in FIGS. 1A-1B. The pitch of the single, helical revolution can be shallow. For example and without limitation, the pitch of the single, helical revolution can be shallow relative to the diameter of the single, helical revolution. In other words, the width *w* of one complete helix turn of the single, helical revolution measured parallel to the axis *a₁* of the helix can be relatively small as compared to the diameter *d* of the single, helical revolution. The axis *a* of the distal portion 16 can be offset from the longitudinal axis *A* of the EP catheter 10. A peripheral portion 17 of the distal portion 16 can be directly coupled to the neck region 18. The distal portion 16 can be configured to deflect in a circular turn at least about 180 degrees relative to the proximal region 14 of the catheter body 12 when the distal portion 16 is in a non-compressed state. A radius *r₁* of the circular turn can be no more than 50 mm.

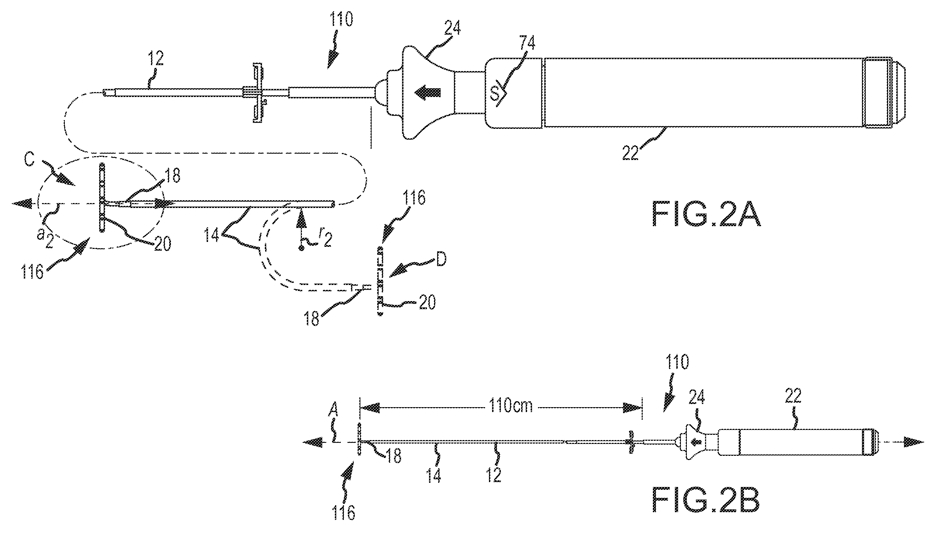

Referring now to FIGS. 2A-2B, an EP catheter 110 according to an embodiment of the present disclosure is generally illustrated. EP catheter 110 is substantially identical to EP catheter 10 described hereinbefore except that the distal portion 116 can be modified and the disposition of the electrodes 20 can also be modified. The distal portion 116 can be located on a distal end of the EP catheter 110 in accordance with the embodiment of the disclosure. However, in accordance with the embodiment of the disclosure, the distal portion 116 comprises a plurality of loops. For example and without limitation, the distal portion 116 can comprise a dual loop. Each of the plurality of loops can be generally circular. While each of the plurality of loops is described as generally circular, they may take any number of shapes in accordance with other embodiments of the disclosure, for example, elliptical. In accordance with the embodiment of the disclosure, the distal portion 116 can be planar as generally illustrated in FIGS. 2A-2B. In other words, a majority of each of the plurality of the loops of distal portion 116 can be disposed within a common plane that is substantially perpendicular to the longitudinal axis *A* of the EP catheter 110. In accordance with the embodiment of the disclosure, the axis *a₂* of the dual loop of the distal portion 116 can be substantially the same as the longitudinal axis *A* of the EP catheter 110. In accordance with the embodiment of the disclosure, at least a portion of the neck region 18 can reside substantially along the longitudinal axis *A* of the EP catheter 110. The distal portion 116 can be configured to deflect in a circular turn at least about 180 degrees relative to the proximal region 14 of the catheter body 12 when the distal portion 16 is in a non-compressed state. A radius *r₂* of the circular turn can be no more than 50 mm in accordance with some embodiments of the disclosure.

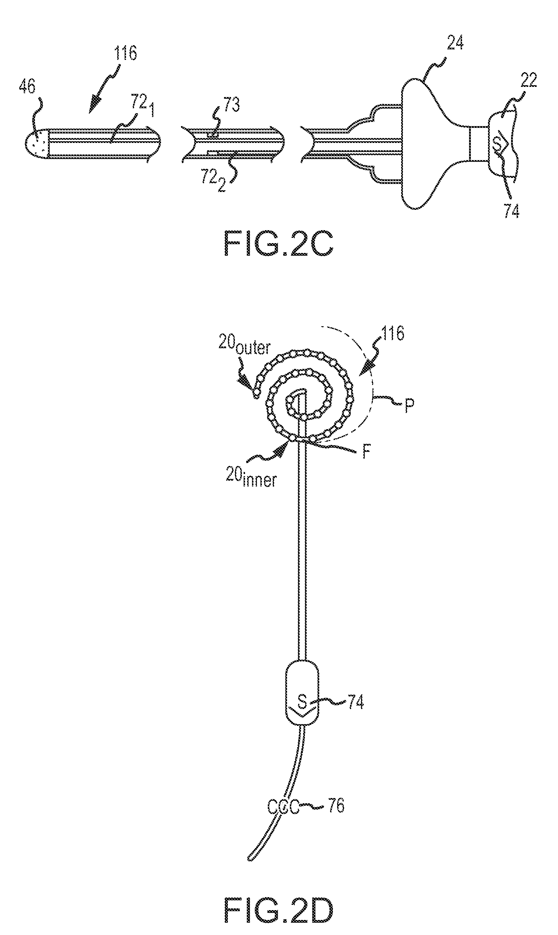

The diameter of the loop of distal portion 16 or the diameter of at least one of the plurality of loops of distal portion 116 can be manually varied in the embodiment of the disclosure. The outermost loop of the plurality of loops of distal portion 116 can be manually varied, while an innermost loop of the plurality of loops of distal portion 116 is of fixed diameter. Although distal portion 116 is generally illustrated as having two loops, namely, an outermost loop and an inner loop, the distal portion 116 can include more than one inner loop in accordance with the embodiment of the disclosure. In accordance with the embodiment of the disclosure, the EP catheter 10, 110 can further include a switch mechanism 74, as generally shown in FIGS. 2A and 2D, for example and without limitation, providing at least two modes of electrical operation. As illustrated in FIG. 2D, in one switch mode, at least one of a plurality of electrodes 20ₒᵤₜₑᵣ connected to the outermost loop of the plurality of loops of distal portion 116 (*i.e.,* a variable loop in some embodiments of the disclosure) can be electrically connected to an EP recording system and/or an electroanatomical localization and visualization system through a cable connector 76 to facilitate pulmonary vein isolation assessment with the outermost loop of the plurality of loops. For example and without limitation, all of the electrodes 20ₒᵤₜₑᵣ connected to the outermost loop of the plurality of loops of distal portion 116 can be electrically connected to the EP recording system and/or electroanatomical localization and visualization system, while none of the electrodes 20ᵢₙₙₑᵣ connected to the innermost loop of the plurality of loops of distal portion 116 are electrically connected to the EP recording system and/or electroanatomical localization and visualization system. The first switch mode can be especially configured to facilitate pulmonary vein isolation assessment because of the variable diameter of the outermost loop of the plurality of loops of distal portion 116. In another switch mode, at least one of a plurality of electrodes 20ₒᵤₜₑᵣ connected to the outermost loop of the plurality of loops of distal portion 116 as well as at least one of the plurality of electrodes 20ᵢₙₙₑᵣ connected to the innermost loop of the plurality of loops of distal portion 116 (*i.e.,* a fixed diameter loop in some embodiments) can be electrically connected to an EP recording system and/or an electroanatomical localization and visualization system through a cable connector 76 to provide high density mapping. For example and without limitation, only a subset of the electrodes 20ₒᵤₜₑᵣ connected to the outermost loop of the plurality of loops of distal portion 116, along with all of the electrodes 20ᵢₙₙₑᵣ connected to the innermost loop of the plurality of loops of distal portion 116 can be electrically connected to the EP recording system and/or electroanatomical localization and visualization system in the second switch mode. In other embodiments, all of the electrodes 20ₒᵤₜₑᵣ connected to the outermost loop of the plurality of loops of distal portion 116, along with all of the electrodes 20ᵢₙₙₑᵣ connected to the innermost loop of the plurality of loops of distal portion 116 can be electrically connected to the EP recording system and/or electroanatomical localization and visualization system in the second switch mode. The second switch mode can be especially configured to enable high density mapping. The switch mechanism 74 can be configured to control the number (*e.g*., all, a subset, none) of the plurality of electrodes 20ᵢₙₙₑᵣ on an inner or the innermost loop of the plurality of loops that are activated and/or the number (*e.g*., all, a subset, none) of the plurality of electrodes 20ₒᵤₜₑᵣ on the outermost loop of the plurality of loops that are activated. The switch mechanism 74 can be manually operated or controlled externally via a software and actuator system. The switch mechanism 74 can be configured to interact with the software in such a way as to utilize the software to select one or more specific electrodes 20 to activate or de-activate. The switch mechanism 74 can be located on the handle 22 in some embodiments of the disclosure.

The diameter of the outermost loop of the plurality of loops of distal portion 116 can be manually varied with one or more tension elements 72 for imparting and releasing tension. FIG 2C illustrates a configuration where a first tension element 72₁ has a distal end coupled to a tip electrode 46 and a proximal end coupled to a suitable actuator 24 on handle 22 and a second tension element 72₂ has a distal end coupled to an anchor 73 disposed within the innermost loop of the plurality of loops of distal portion 116 and a proximal end coupled to a suitable actuator 24 on handle 22. Such tension elements 72 can couple to structure within one or more locations within the distal portion 16, 116 (*e.g*., using para-aramid synthetic fibers such as those sold under the brand name KEVLAR® available from E.I. du Pont de Nemours and Company, metallic or composite wires or axially rigid elements, thin pull wires, flat or round wires, and the like). In those embodiments of the disclosure that allow for adjustment of the diameter of the loop of distal portion 16 and/or at least one of the plurality of loops of distal portion 116, the handle 22 can be configured for adjusting the diameter of one or more portions of the loop(s) of distal portion 16, 116. In particular, the handle 22 can include one or more tension elements 72 operably coupled to adjust at least a portion of the loop(s) of distal portion 16, 116 (*e.g*., terminating at an anchor member disposed within one or more locations of the loop(s)). The EP catheter 10, 110 can also include other elements adapted to alter a radius of curvature of at least part of the loop(s) (e*.g.,* to provide a complex radius, or knuckle-like cross section to the loop(s)). The tension elements 72 can be configured to adjust the loop(s) of the distal portion 16, 116 in a common plane or in different planes in accordance with various embodiments of the disclosure. The tension elements 72 may allow an operator of EP catheter 10, 110 to adjust the diameter of the loop(s) of distal portion 16, 116 to conform generally to the interior diameter of a portion of the subject's adjacent anatomy. The tension elements 72 can be coupled to the loop(s) of the distal portion 16, 116 at one end and to a suitable actuator on handle 22 at a second opposing end. Still other mechanisms for varying the radius or radii of curvature of the distal portion 16, 116 are described in U.S. Patent No. 7,606,609, issued October 20, 2009. A fulcrum point F where the variable diameter of the outermost loop can begin, as generally illustrated in FIG. 2D, can be contained within the distal portion 16, 116. Still referring to FIG. 2D, an exemplary path P of the outermost loop having a variable diameter or radius is generally illustrated by dotted lines.

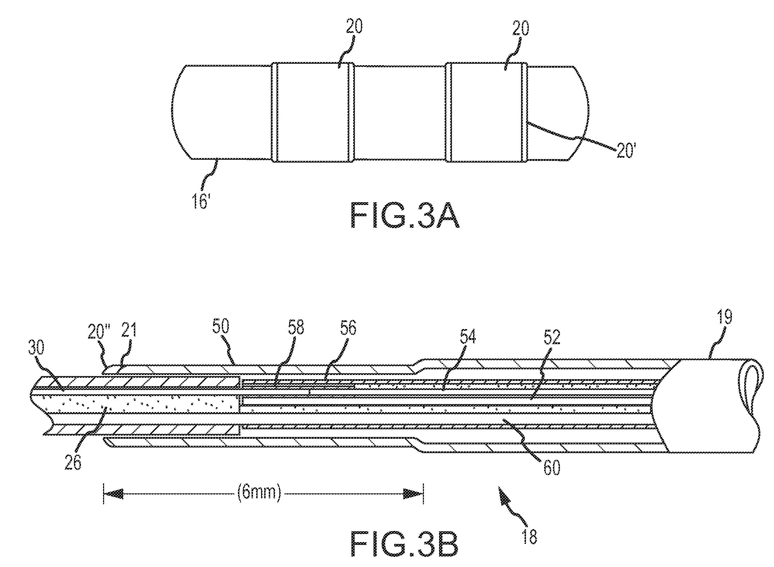

FIG. 3A is an enlarged view of a segment 16' of the distal portion 16, 116 of the exemplary EP catheters 10, 110 of FIGS. 1A and 2A. In particular, FIG. 3A is an illustration of a pair of electrodes 20 residing on a segment 16' of the distal portion 16, 116. Electrodes 20 can be ring-type electrodes or any other electrodes suitable for a particular application of EP catheter 10, 110 in accordance with various embodiments of the disclosure. For example, where EP catheter 10, 110 is intended for use in a contactless EP study, electrodes 20 can be configured as described in United States application no. 12/496,855, filed 2 July 2009, now published as United States patent application publication no. 2011/0004087. In addition to serving sensing purposes (*e.g*., cardiac mapping and/or diagnosis), electrodes 20 can be employed for therapeutic purposes (*e.g*., cardiac ablation and/or pacing).

At least the lateral edges 20' of electrodes 20 can be bonded to the adjacent relatively smaller (*e.g.,* 4F) diameter biocompatible tubing (*e.g.,* PTFE or the like) of the distal portion 16, 116 with a biocompatible material such as a polyurethane matrix composed of POLYCIN® 936 and VORITE™ 689 (mixed 52:48 percent, as an example) produced by CasChem Inc. of Bayonne, New Jersey. Although this biocompatible material is mentioned in particular, other types of biocompatible materials, including other polymers, can be used in accordance with other embodiments of the disclosure.

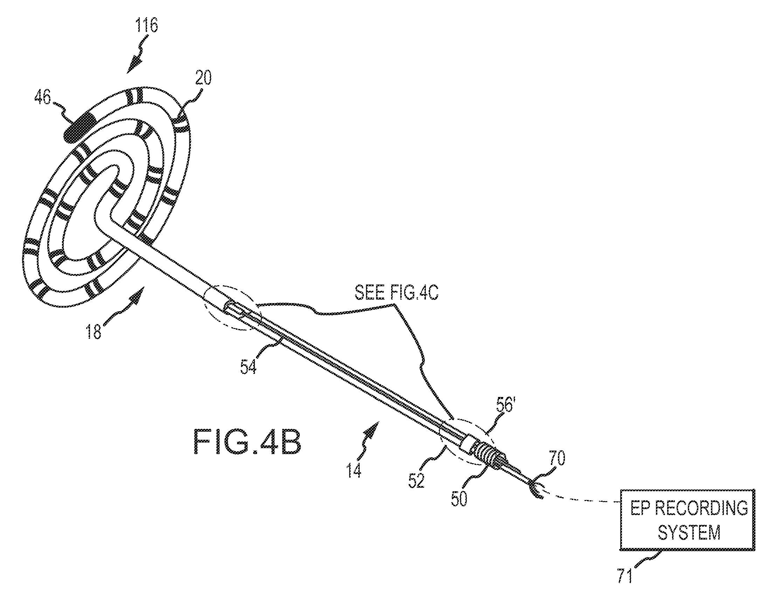

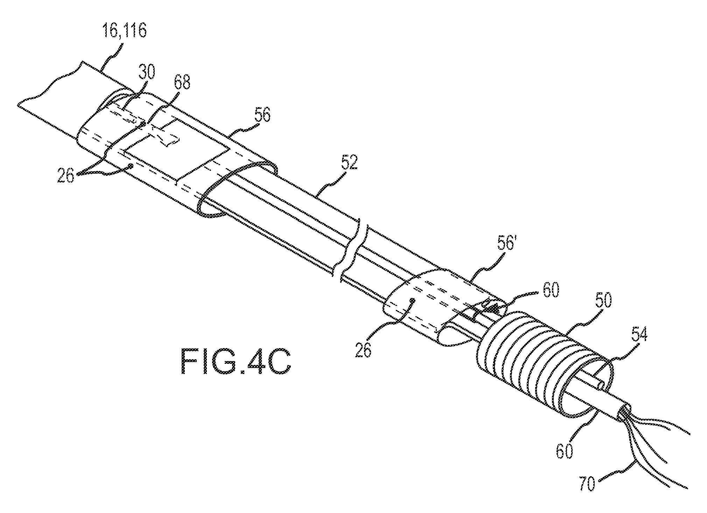

FIG. 3B is an elevational side view in partial cross section of a neck portion 18 formed just proximal of the distal portion 16, 116 of the exemplary EP catheters 10, 110 depicted in FIGS. 1A, 1B, 2A, and 2B. As shown, an extended braid tube/spring assembly 50 surrounds a variety of subcomponents of catheter 10, 110 and is itself wrapped by biocompatible tubing 19 that covers the neck region 18 and transitions the outer diameter to the about 4F distal portion 16, 116. The extended braid tube/spring assembly 50 can include at least a single layer metallic braid core wrapped in the biocompatible tubing 19. Where the extended braid tube/spring assembly 50 terminates at its distal edge 21, a small amount of medical grade adhesive polymer 20" (*e.g.,* like the biocompatible material used at the edges 20' of electrodes 20) can be applied. A polyimide tube 56 can pass through the extended braid tube/spring assembly 50 (and neck region 18) and into the distal portion 16, 116 and isolates a plurality of elongate conductive wires 70 (shown in FIG. 4C, for example) that couple the electrodes 20 to remote circuitry via a handle (22 as shown in FIGS. 1A, 1B, 2A, and 2B) having a mass termination where the conductors 70 pass through the handle 22 to couple to an EP recording system 71 or other diagnostic equipment, for example.

Handle 22 can be coupled to the proximal region 14 of the catheter body 12. Handle 22 can include a means for imparting tension to an activation wire 54. For example and without limitation, the means for imparting tension to the activation wire 54 can comprise a flat wire subassembly 52. Although a flat wire subassembly 52 is mentioned in detail, the means for imparting tension to the activation wire 54 can be another element such as a round wire, a thread of fiber such as a para-aramid synthetic fiber including those sold under the brand name KEVLAR® available from E. I. du Pont de Nemours and Company, and the like. The activation wire 54 can be actuated in order to deflect the proximal region 14 of catheter body 12 such that the distal portion 16, 116 is oriented generally towards the pulmonary vein ostium of interest. Electrodes 20 can then be employed for diagnostic or therapeutic purposes.

A flat wire assembly 52 coupled to the activation wire 54 is adapted to impart and release tension to deflect the distal portion 16, 116 in a plane parallel to a longitudinal axis of the flat wire assembly 52 (via manipulation of the handle 22, such as by rotation or linear actuation members, and the like). The flat wire assembly 52 is sometimes described as a planarity member or element because it promotes such planar deflection. A short segment of polyimide tubing 56 surrounds a junction of several components; namely, a lubricous tubing member 58 (*e.g*., PEEK tubing) that receives a proximal end of an elongate shape memory member 30 (formed of nitinol, for example) that is preformed into a desired dimension and configuration for distal portion 16, 116. The shape memory member 30 can extend through at least a portion of the distal portion 16, 116, thereby shaping at least a portion of the distal portion into a plurality of loops disposed within a common plane, each of the plurality of loops having a predefined radius of curvature in accordance with the embodiment of the disclosure. The shape memory member 30 can be configured to promote a predefined radius of curvature of the distal portion 16, 116. The shape memory member 30 can comprise a nickel titanium material or an alloy thereof.

The activation wire 54 coupled to the flat wire subassembly 52 is configured to transfer forces to the catheter shaft proximal of the distal portion 16, 116 and the neck region 18 via an actuation mechanism. A proximal end of the activation wire 54 can be coupled to the actuation mechanism. The actuation mechanism can comprise a push-pull knob 24 as depicted in FIGS. 1A-1B and 2A-2B in accordance with embodiments of the disclosure. Although a push-pull knob 24 is mentioned and illustrated in detail, other actuation mechanisms can be used in various embodiments of the disclosure. For example and without limitation, a rotary knob or any other structure known to those of ordinary skill in the art to be configured to actuate the activation wire 54 can be used in accordance with various embodiments of the disclosure. The actuation mechanism can be operably coupled to the activation wire 54. Various handles and their associated actuators for use in connection with deflecting EP catheters are known, and thus, handle 22 will not be described in further detail herein.

In the embodiment of the present invention, the distal portion 116 has an overall outer diameter of about 20 mm (*i.e.,* for the outermost loop) with a 4F dimension for portion 116 and 1 mm (wide or axial length) platinum electrodes 20 and a 2 mm (long or axial length) tip electrode 46. The outermost diameter of the outermost loop of the plurality of loops of the distal portion 116 can be between about 10 mm and about 40 mm in various embodiments of the disclosure. For example and without limitation, the outermost diameter of the outermost loop of the plurality of loops can be about 20 mm. In the embodiment of the disclosure, the electrodes can be substantially evenly spaced on the distal portion 116. For example and without limitation, the electrodes 20 can be spaced about 4 mm apart, about 6.5 mm apart, or any other nominal spacing between them. Although this spacing is mentioned in detail, any other spacing arrangement can be used in accordance with other embodiments. In the embodiment of the disclosure, the electrodes can be disposed in a substantially bi-polar spacing arrangement having a plurality of pairs of electrodes 20. Each of the plurality of pairs of electrodes can be spaced about 2.5 mm apart, and the electrodes within each of the plurality of pairs of electrodes can be spaced about 1 mm apart. Of course, the electrode spacing can vary. In the embodiment of the disclosure, the spacing between the tip electrode 46 to the most distal ring-type electrode 20 can be about 1 mm or other value. Generally, each pair can be equally separated from each other pair about 2.5 mm to about 7 mm apart, although the spacing can be greater or less in various embodiments. In the embodiments depicted herein, the diameter of the outermost loop of the plurality of loops of the distal portion 116 is fixed (*e.g.,* at about 20 mm to about 33, or more or less, if desired)

At the junction of the flat wire subassembly 52 with the nitinol wire 30 wrapped in, for example, PEEK tubing 58, urethane adhesive (denoted by reference numeral 26 in FIG. 4C, for example) can be applied between, above, and around the components within the polyimide tubing 56 to encapsulate same. Similarly, urethane adhesive 26 can be impregnated into the interstices of the neck region 18 and the distal portion 16, 116 to reduce or eliminate any migration of the nitinol wire 30 or PEEK tubing 58 or polyimide tube 60 (surrounding conductors 70) during use.

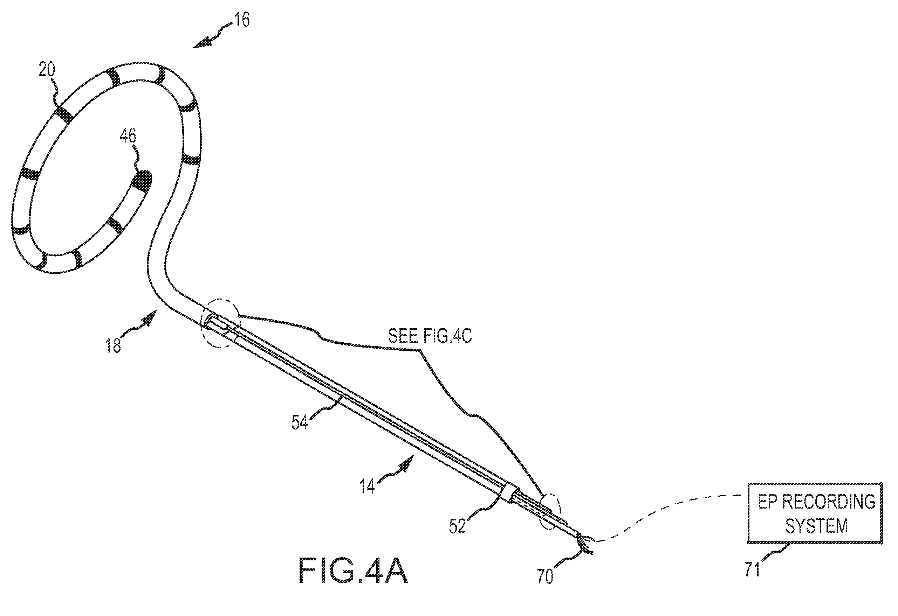

Referring now to FIGS. 4A-4B, close up isometric views of the distal portions 16, 116 of the exemplary EP catheters 10, 110 of FIGS. 1A, 1B and 2A, 2B, respectively (with a view of connecting elements within interior portions of the catheter body 12) are illustrated. As illustrated, the proximal and distal ends of the flat wire subassembly 52 (*e.g.,* implemented to promote planarity during deflection) are emphasized.

FIG. 4C is an enlarged isometric fragmented view of the interior details of the ends of the various connecting elements within the interior of the proximal region 14 and the neck region 18 of the catheter body 12 of FIGS. 4A-4B. As depicted, the proximal end of a flattened PEEK tube 68 that contains the nitinol wire 30 is adhered with urethane adhesive 26 (or other suitable medical grade adhesive) to the flat wire subassembly 52 and wrapped in polyimide tubing 56 for containment. The proximal end of the flat wire subassembly 52 couples via a segment of polyimide tubing 56' filled with urethane adhesive 26 that also encapsulates the smaller diameter polyimide tubing 60 where the activation wire 54 resides. A gap of about 1-2 mm between the tubing 56' and the distal end of extended braid/spring subassembly 50 should be optionally maintained (as depicted) and the activation wire 54 and conductor wires 70 are conveyed through extended braid/spring subassembly 50 to a handle 22 or other remote location.

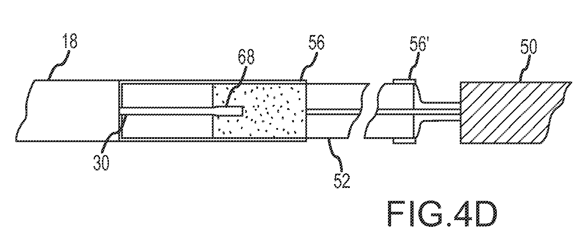

FIG. 4D is an enlarged fragmented plan view of the interior details of the ends of the connecting elements within the interior of catheter body 12 shown in FIGS. 4A-4B. As depicted, the flattened section of the PEEK tubing 68 disposed within the polyimide tubing 56 can comprise a 1 mm segment to promote adhesion to the urethane adhesive 26 impregnated therein and thus to the flat wire subassembly 52. Similarly, the proximal end of the flat wire subassembly 52 can be surrounded by polyimide tubing 56' and impregnated with urethane adhesive 26 to promote mechanical coupling to the adjacent extended braid/spring subassembly 50. A suitable biocompatible compound (*e.g*., such as the biocompatible material used at the edges 20' of electrodes 20 or the medical grade adhesive polymer 20" applied at the distal edge 21 of the extended braid tube/spring assembly 50) can be applied to the junction between the outer covering for distal portion 16, 116 and the neck region 18.

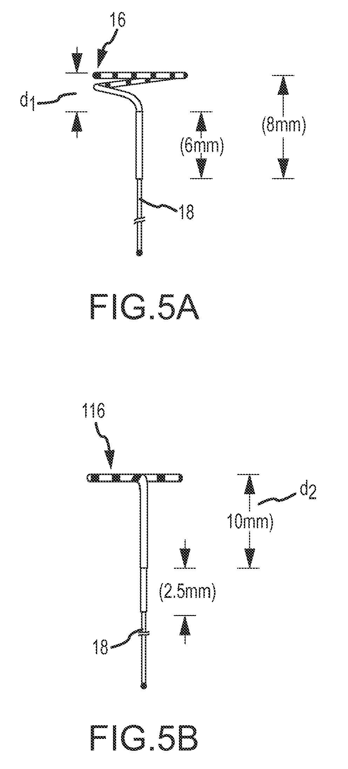

FIG. 5A is an elevational view showing exemplary dimensions of the distal portion 16 of the exemplary EP catheter 10 of FIGS. 1A and 1B. At the perspective illustrated, the off-axis junction with the catheter body 12 is not apparent. For example and without limitation, the distance *d₁* from the distal end of the distal portion 16 to the neck region 18 can be on the order of about 2 mm. Although this distance is mentioned in detail, other dimensions can be used. Whatever dimension is used for the distance *d₁* for the distal portion 16, the wire support length therefrom should be a reasonable corresponding length.

FIG. 5B is an elevational view showing exemplary dimensions of the distal portion 116 of the exemplary EP catheter 110 of FIGS. 2A and 2B in accordance with the embodiment of the disclosure. For example and without limitation, the distance *d₂* from the distal end of the distal portion 116 in accordance with the embodiment of the disclosure to the neck region 18 can be on the order of about 10 mm. Although this distance is mentioned in detail, other dimensions can be used in accordance with various embodiments of the disclosure. Whatever dimension is used for the distance *d₂* for the distal portion 116, the wire support length therefrom should be a reasonable corresponding length.

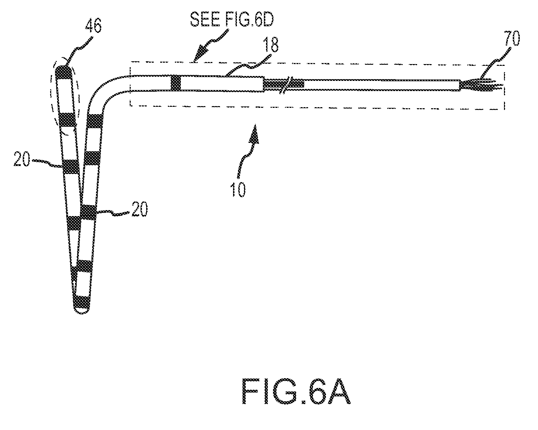

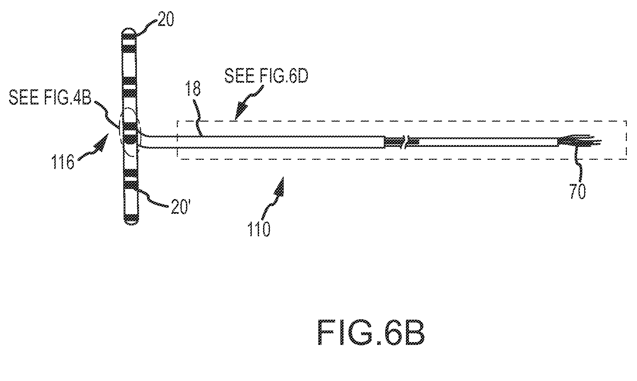

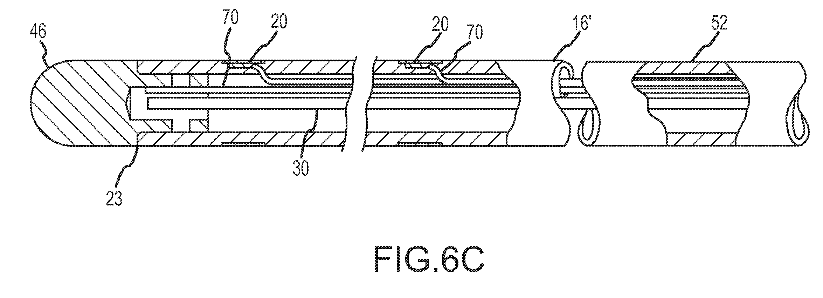

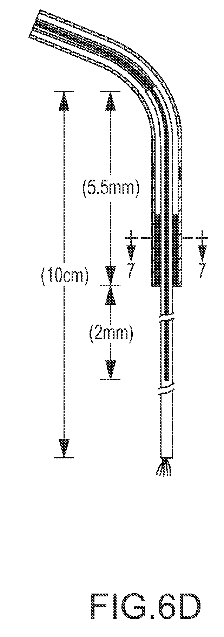

FIG. 6A depicts the distal portion 16 of the exemplary EP catheter 10 of FIGS. 1A and 1B (with cross references to details shown in FIGS. 6C and 6D). The distal portion 16 includes substantially evenly-spaced (*i.e.,* with respect to shaft length) ten-pole electrodes 20, 46 with a separation between adjacent electrodes 20, 46. Although these particular electrodes are mentioned in detail, various dimensions can be used for the electrodes 20. In addition, although the electrodes are described as evenly spaced, the spacing between electrodes 20, 46 can vary. FIG. 6B depicts the distal portion 116 of the exemplary EP catheter 110 of FIGS. 2A and 2B (with cross reference to details shown in FIGS. 6C and 6D). In the illustrated embodiment, the distal portion 116 includes paired twenty-pole electrodes 20, 46 with a nominal separation between adjacent electrodes 20, 46 making up a pair of electrodes. For example and without limitation, the nominal separation between adjacent electrodes 20 making up a pair of electrodes 20 can be about 1 mm. For example and without limitation, the separation between adjacent pairs of electrodes 20 can be about 2.5 mm. Although these particular spacing configurations are mentioned in detail, various dimensions can be used for the electrodes 20 and the spacing therebetween. At the proximal end of the catheter body 12 (not specifically shown), a plurality of individually electrically insulated elongate conductors 70 emerge and are adapted to be individually coupled to a mass termination terminal within a handle 22 for ultimate electrical communication with an EP recording system 71 and/or an electroanatomical localization and visualization system (*e.g*., such as the ENSITE® system of St. Jude Medical, Inc. operating the ONEMAP facility or other similar systems for monitoring cardiac activity and providing one or more visual representations of same).

FIG. 6C is an enlarged fragmentary view in partial cross section and partial cut-away of the distal tip electrode 46 and two ring-type electrodes 20 and flat wire subassembly 52 connection within the catheter body 12, respectively, shown in FIG. 6A or 6B. Each electrode 20, 46 couples via an elongate conductor 70 in FIG. 6C to remote EP recording system 71 and/or electroanatomical localization and visualization equipment (*e.g*., such as the ENSITE® system of St. Jude Medical, Inc.). A biocompatible adhesive 23 (*e.g*., LOCTITE® adhesive) can be applied to the junction of the biocompatible tubing of the distal portion 16, 116 and the electrode 46 to eliminate body fluid ingress therein. A so-called safety wire or element (not shown) can couple to the electrode 46 and a proximal location to reduce or eliminate the chance that the electrode 46 might separate from the catheter assembly 10.

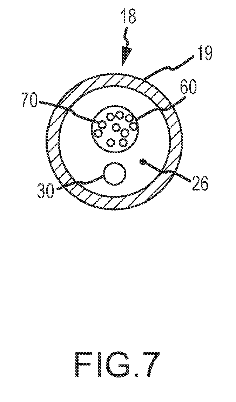

FIG. 6D is an enlarged fragmentary view in partial cross section of the catheter body 12 near the neck region 18 shown in FIGS. 6A and 6B and indicates a cross sectional view along lines 7-7 therein which is reflected in FIG. 7 hereinbelow described. The dimensions indicated in FIG. 6D are merely exemplary and illustrative and not intended as limiting in any way. Although these dimensions are illustrated in detail, the dimensions for the neck region 18 of the catheter body 12 can vary in accordance with various embodiments of the disclosures.

FIG. 7 is a cross-sectional view of the EP catheter 10 illustrated in FIG. 6D taken along line 7-7 as shown in FIG. 6D. The biocompatible tubing 19 overlaying neck region 18 includes conductor wires 70 (for electrodes 20, 46) surrounded by polyimide tubing 60 and nominally spaced from nitinol wire 30 by a space impregnated with urethane adhesive 26. A cross-sectional view of the EP catheter 110 taken along line 7-7 as shown in FIG. 6D would include additional conductor wires 70 (for electrodes 20, 46) surrounded by polyimide tubing 60.

The method of manufacturing EP catheters 10, 110 having a proximal region 14, a neck region 18, and a distal portion 16, 116 can include manufacturing an EP catheter 110 having a distal portion 116 being predisposed into a plurality of loops when in a non-compressed state, wherein a majority of each of the plurality of loops is disposed within a common plane. The method can include the step of forming a shape wire or shape memory structure 30 into a plurality of loops, wherein a majority of each of the plurality of loops is disposed in a common plane.

The above method can include the additional steps of soldering the shape wire or shape memory structure 30 and an electrical conducting wire 70 to an interior of a tip electrode 46; covering the shape wire or shape memory structure 30 and the electrical conducting wire 70 with a biocompatible polymer 19 to form a distal portion 16, 116 and the neck region 18 of the EP catheter 10, 110; connecting a plurality of ring-type electrodes 20 to the distal portion 16, 116; and coupling the plurality of ring-type electrodes 20 to the electrical conducting wire 70. The method can further include the steps of adhering a lubricious tube 58 to a segment of flat wire 52; threading an activation wire 54 through the lubricious tube 58; coupling a distal end of the segment of flat wire 52 to the neck region 18; and coupling a proximal end of the activation wire 54 to an actuation mechanism (e.g., push-pull handle or knob 24). The method can further include the steps of covering the activation wire 54 and the segment of flat wire 52 with an extended braid assembly 50, the extended braid assembly 50 comprising at least a single layer metallic braid core wrapped in biocompatible outer layer 19; and coupling a distal end of the outer layer 19 to the neck region 18. The biocompatible outer layer 19 can have a first segment having a first durometer and a second segment having a second durometer. The first segment can be proximal of the second segment, and the first durometer can be greater than the second durometer.

Although an embodiment of this disclosure has been described above with a certain degree of particularity, those skilled in the art could make numerous alterations to the disclosed embodiment without departing from the scope of this invention. All directional references (*e.g*., upper, lower, upward, downward, left, right, leftward, rightward, top, bottom, above, below, vertical, horizontal, clockwise, and counterclockwise) are only used for identification purposes to aid the reader's understanding of the present invention, and do not create limitations, particularly as to the position, orientation, or use of the invention. Joinder references (*e.g*., attached, coupled, connected, and the like) are to be construed broadly and may include intermediate members between a connection of elements and relative movement between elements. As such, joinder references do not necessarily infer that two elements are directly connected and in fixed relation to each other. It is intended that all matter contained in the above description or shown in the accompanying drawings shall be interpreted as illustrative only and not limiting. Changes in detail or structure may be made without departing from the scope of the invention as defined in the appended claims.

## Claims

1. An electrophysiology (EP) catheter (10, 110) having a longitudinal axis (A), the EP catheter comprising:
a catheter body (12) having:
a proximal region (14);
a neck region (18) coupled to the proximal region (14); and
a distal portion (16, 116) coupled to the neck region (18), the distal portion (16, 116) being predisposed into a plurality of loops comprising at least a first loop and a second loop when in a non-compressed state, wherein a majority of the first loop and a majority of the second loop is disposed within a common plane that is substantially perpendicular to the longitudinal axis (A) of the EP catheter (10, 110); and
a plurality of electrodes (20) coupled to the distal portion (16, 116),
further including at least one tension element (72) coupled to at least one anchor member within the distal portion (16, 116) and coupled to an actuator in a handle (22) configured to alter a diameter of an outermost loop of the plurality of loops, **characterised in that** the outermost loop of the plurality of loops is configured to provide a variable diameter and wherein an innermost loop of the plurality of loops is configured to provide a fixed diameter or radius of curvature.

2. The EP catheter (10, 110) according to claim 1, wherein the outermost loop of the plurality of loops includes a plurality of electrodes (20 outer) and wherein the innermost loop of the plurality of loops includes a plurality of electrodes (20 inner), and wherein the EP catheter further includes at least one switch mechanism (74) configured to control the number of the plurality of electrodes (20 outer) on the outermost loop that are activated and the number of the plurality of electrodes (20 inner) on the innermost loop that are activated.

3. The EP catheter (10, 110) according to claim 1 or 2, wherein the distal portion (16, 116) is configured to deflect in a circular turn at least about 180 degrees relative to the proximal region of the catheter body (12) when the distal portion is in a non-compressed state.

4. The EP catheter (10, 110) according to any one of the preceding claims, wherein the distal portion (16, 116) includes a shape memory material comprising a nickel titanium material or an alloy thereof.

5. The EP catheter (10, 110) according to claim 4, wherein the shape memory material is configured to shape at least a portion of the distal portion into first loop and the second loop disposed within a common plane, each of the first loop and the second loop having a predefined radius of curvature.

6. The EP catheter (10, 110) according to any one of the preceding claims, wherein at least one of the plurality of electrodes (20) is coupled to a distal tip portion of one of the plurality of loops.

7. The EP catheter (10, 110) according to any one of the preceding claims, further comprising a braided structure (50) embedded near a junction of the neck region (18) and the distal portion (16, 116).

8. The EP catheter (10, 110) according to any one of the preceding claims, further comprising a handle (22) and an activation wire (54) extending from the handle, wherein the handle (22) includes means for imparting tension to the activation wire (54) such that forces acting on the activation wire are transmitted to the proximal region (14) in order to deflect the catheter body (12).

9. The EP catheter (10, 110) according to claim 8, wherein the handle further comprises a mass electrical termination structure for receiving a plurality of electrical conductors.

10. The EP catheter (10, 110) according to claim 8 or 9, further comprising a flat wire subassembly (52) disposed between the handle (22) and the distal portion (16, 116).

## Patentansprüche

1. Elektrophysiologie (EP)-Katheter (10, 110) mit einer Längsachse (A), wobei der EP-Katheter umfasst:
einen Katheterkörper (12), der
einen proximalen Bereich (14),
einen Halsbereich (18), der mit dem proximalen Bereich (14) gekoppelt ist, und einen distalen Abschnitt (16, 116), der mit dem Halsbereich (18) gekoppelt ist, aufweist, wobei der distale Abschnitt (16, 116) im Vorhinein als eine Mehrzahl von Schlingen ausgebildet ist, die mindestens eine erste Schlinge und eine zweite Schlinge umfassen, wenn er sich in einem nicht-zusammengedrückten Zustand befindet, wobei ein Hauptteil der ersten Schlinge und ein Hauptteil der zweiten Schlinge innerhalb einer gemeinsamen Ebene angeordnet sind, die im Wesentlichen senkrecht zu der Längsachse (A) des EP-Katheters (10, 110) ist, und
eine Mehrzahl von Elektroden (20), die an den distalen Abschnitt (16, 116) gekoppelt sind,
ferner umfassend mindestens ein Spannelement (72), das an mindestens ein Verankerungselement innerhalb des distalen Abschnitts (16, 116) gekoppelt ist und an einen Aktuator in einem Griff (22) gekoppelt ist, der zum Verändern des Durchmessers einer äußersten Schlinge der Mehrzahl von Schlingen ausgebildet ist, **dadurch gekennzeichnet, dass** die äußerste Schlinge der Mehrzahl von Schlingen so ausgebildet ist, dass sie einen variablen Durchmesser bereitstellt, und wobei eine innerste Schlinge der Mehrzahl von Schlingen so ausgebildet ist, dass sie einen feststehenden Durchmesser oder Krümmungsradius bereitstellt.

2. EP-Katheter (10, 110) nach Anspruch 1, bei dem die äußerste Schlinge der Mehrzahl von Schlingen eine Mehrzahl von Elektroden (20 äußere) umfasst und wobei die innerste Schlinge der Mehrzahl von Schlingen eine Mehrzahl von Elektroden (20 innere) umfasst und wobei der EP-Katheter ferner mindestens einen Schaltmechanismus (74) umfasst, der so ausgebildet ist, dass er die Anzahl der Mehrzahl von Elektroden (20 äußere) an der äußersten Schlinge, die aktiviert sind, und die Anzahl der Mehrzahl von Elektroden (20 innere) an der innersten Schlinge, die aktiviert sind, steuert.

3. EP-Katheter (10, 110) nach Anspruch 1 oder 2, bei dem der distale Abschnitt (16, 116) so ausgebildet ist, dass er in einer kreisförmigen Windung um mindestens etwa 180 Grad bezogen auf den proximalen Bereich des Katheterkörpers (12) ausgelenkt wird, wenn sich der distale Abschnitt in einem nicht-zusammengedrückten Zustand befindet.

4. EP-Katheter (10, 110) nach einem der vorhergehenden Ansprüche, bei dem der distale Abschnitt (16, 116) ein Formgedächtnismaterial umfasst, das ein Nickel-Titan-Material oder eine Legierung davon umfasst.

5. EP-Katheter (10, 110) nach Anspruch 4, bei dem das Formgedächtnismaterial so ausgebildet ist, dass es mindestens einen Abschnitt des distalen Abschnitts zu einer ersten Schlinge formt und die zweite Schlinge innerhalb einer gemeinsamen Ebene angeordnet ist, wobei jede der ersten Schlinge und der zweiten Schlinge einen vorgegebenen Krümmungsradius aufweist.

6. EP-Katheter (10, 110) nach einem der vorhergehenden Ansprüche, bei dem mindestens eine der Mehrzahl von Elektroden (20) an einen distalen Spitzenabschnitt von einer der Mehrzahl von Schlingen gekoppelt ist.

7. EP-Katheter (10, 110) nach einem der vorhergehenden Ansprüche, der ferner eine geflochtene Struktur (50) umfasst, die in der Nähe einer Verbindungsstelle des Halsbereichs (18) und des distalen Abschnitts (16, 116) eingebettet ist.

8. EP-Katheter (10, 110) nach einem der vorhergehenden Ansprüche, der ferner einen Griff (22) und einen Aktivierungsdraht (54) umfasst, der sich von dem Griff erstreckt, wobei der Griff (22) Mittel zum Spannen des Aktivierungsdrahts (54) in einer Weise, dass Kräfte, die auf den Aktivierungsdraht wirken, auf den proximalen Bereich (14) zum Auslenken des Katheters (12) übertragen werden, umfasst.

9. EP-Katheter (10, 110) nach Anspruch 8, bei dem der Griff ferner eine elektrische Masse-Endstruktur zum Aufnehmen einer Mehrzahl von elektrischen Leitern umfasst.

10. EP-Katheter (10, 110) nach Anspruch 8 oder 9, der ferner eine flache Drahtteilanordnung (52) umfasst, die zwischen dem Griff (22) und dem distalen Abschnitt (16, 116) angeordnet ist.

## Revendications

1. Cathéter d'électrophysiologie (EP) (10, 110), présentant un axe longitudinal (A), le cathéter EP comprenant :
un corps de cathéter (12) présentant :
une région proximale (14) ;
une région de col (18) couplée à la région proximale (14) ; et
une partie distale (16, 116) couplée à la région de col (18), la partie distale (16, 116) étant préalablement disposée en une pluralité de boucles comprenant au moins une première boucle et une deuxième boucle lorsqu'elle se trouve dans un état non comprimé, dans lequel une majeure partie de la première boucle et une majeure partie de la deuxième boucle sont disposées à l'intérieur d'un plan commun qui est sensiblement perpendiculaire à l'axe longitudinal (A) du cathéter EP (10, 110) ; et
une pluralité d'électrodes (20) couplées à la partie distale (16, 116),
comprenant en outre au moins un élément de tension (72) couplé à au moins un élément d'ancrage à l'intérieur de la partie distale (16, 116) et couplé à un actionneur dans un manche (22) configuré pour modifier un diamètre d'une boucle extérieure extrême de la pluralité de boucles,
**caractérisé en ce que** la boucle extérieure extrême de la pluralité de boucles est configurée de manière à offrir un diamètre variable, et dans lequel une boucle intérieure extrême de la pluralité de boucles est configurée de manière à offrir un diamètre ou un rayon de courbure fixe.

2. Cathéter EP (10, 110) selon la revendication 1, dans lequel la boucle extérieure extrême de la pluralité de boucles comprend une pluralité d'électrodes (20ₒᵤₜₑᵣ), et dans lequel la boucle intérieure extrême de la pluralité de boucles comprend une pluralité d'électrodes (20ᵢₙₙₑᵣ), et dans lequel le cathéter EP comprend en outre au moins un mécanisme de commutation (74) configuré de manière à commander le nombre de la pluralité d'électrodes (20ₒᵤₜₑᵣ) sur la boucle extérieure extrême qui sont activées et le nombre de la pluralité d'électrodes (20ᵢₙₙₑᵣ) sur la boucle intérieure extrême qui sont activées.

3. Cathéter EP (10, 110) selon la revendication 1 ou 2, dans lequel la partie distale (16, 116) est configurée de manière à se défléchir en une spire circulaire d'au moins environ 180 degrés par rapport à la région proximale du corps de cathéter (12) lorsque la partie distale se trouve dans un état non comprimé.

4. Cathéter EP (10, 110) selon l'une quelconque des revendications précédentes, dans lequel la partie distale (16, 116) comprend un matériau à mémoire de forme comprenant un matériau de nickel titane ou un alliage de celui-ci.

5. Cathéter EP (10, 110) selon la revendication 4, dans lequel le matériau à mémoire de forme est configuré de manière à configurer au moins une partie de la partie distale en la première boucle et la deuxième boucle disposées à l'intérieur d'un plan commun, chacune de la première boucle et de la deuxième boucle présentant un rayon de courbure prédéfini.

6. Cathéter EP (10, 110) selon l'une quelconque des revendications précédentes, dans lequel au moins une de la pluralité d'électrodes (20) est couplée à une partie de pointe distale de l'une de la pluralité de boucles.

7. Cathéter EP (10, 110) selon l'une quelconque des revendications précédentes, comprenant en outre une structure tressée (50) incorporée à proximité d'une jonction de la région de col (18) et de la partie distale (16, 116).

8. Cathéter EP (10, 110) selon l'une quelconque des revendications précédentes, comprenant en outre un manche (22) et un fil d'activation (54) qui s'étend à partir du manche, dans lequel le manche (22) comprend des moyens pour imprimer une tension au fil d'activation (54) de telle sorte que des forces qui agissent sur le fil d'activation soient transmises à la région proximale (14) dans le but de défléchir le corps de cathéter (12).

9. Cathéter EP (10, 110) selon la revendication 8, dans lequel le manche comprend en outre une structure de terminaison électrique de masse pour recevoir une pluralité de conducteurs électriques.

10. Cathéter EP (10, 110) selon la revendication 8 ou 9, comprenant en outre un sous-ensemble de fils plats (52) disposé entre le manche (22) et la partie distale (16, 116).
